# FASCICULE DE BREVET EUROPEEN

(11) **EP 2 791 124 B1**
(45) Date de publication et mention de la délivrance du brevet: **07.06.2017**
(21) Numéro de dépôt: 12809186.5
(22) Date de dépôt: 13.12.2012
(51) Int. Cl.: C07D 307/24, C07D 307/66, C07D 307/14, C07D 307/68

(54) **PROCEDE DE FABRICATION DE COMPOSES COMPRENANT DES FONCTIONS NITRILES**
VERFAHREN ZUR HERSTELLUNG VON VERBINDUNGEN MIT NITRILFUNKTIONEN
METHOD FOR MANUFACTURING COMPOUNDS INCLUDING NITRILE FUNCTIONS

(30) Priorité: 16.12.2011 FR 1161823
(43) Date de publication de la demande: 22.10.2014
(73) Titulaire: Rhodia Operations, 93306 Aubervilliers (FR)
(72) Inventeur: JACQUOT, Roland, F-69340 Francheville (FR); MARION, Philippe, F-69390 Vernaison (FR)
(74) Mandataire: Chatelan, Florence Anne
(86) Numéro de dépôt international: PCT/EP2012/075377
(87) Numéro de publication internationale: WO 2013/087765

(56) Documents cités:
- WO-A1-2010/063632
- US-A- 4 743 702
- US-A1- 2005 059 836
- US-A1- 2005 187 266

## Description

La présente invention concerne la fabrication de composés comprenant des fonctions nitriles.

Elle se rapporte plus particulièrement à la fabrication de composés comprenant des fonctions nitriles à partir de composés comprenant des fonctions carboxyliques, avantageusement d'origine naturelle et renouvelable.

Les composés comprenant des fonctions nitriles sont des produits importants pour la fabrication de composés amines. Les composés dinitriles conduisent à des amines qui sont par exemple des monomères à l'origine de polymères tels que le polyamide. par exemple. Les composés mononitriles conduisent à des amines ou à des amides qui sont par exemple utilisés pour la fabrication de tensioactifs cationiques.

De nombreux procédés de synthèse de nitriles ont été proposés, notamment des procédés de synthèse à partir d'ammoniac et d'acides carboxyliques. Ces procédés utilisent principalement comme matière première de départ des composés hydrocarbures issus du raffinage du pétrole.

Compte tenu de l'épuisement de la ressource pétrolière, de nombreux travaux de recherche sont entrepris pour développer des procédés de synthèse de ces composés, importants pour la fabrication de matériaux utilisés dans de nombreuses applications, à partir de matières premières ou ressources dites renouvelables, ou à partir de matières premières recyclées, qui sont habituellement détruites ou valorisées sous forme d'énergie. Ces ressources renouvelables peuvent être obtenues à partir de matière végétale cultivée ou non telle que les arbres, les plantes comme la canne à sucre, le maïs, le manioc, le blé, le colza, le tournesol, la palme, le ricin ou analogues.

Cette matière végétale est transformée par des procédés comprenant généralement plusieurs étapes mécaniques, chimiques et biologiques.

L'acide 2,5-furannedicarboxylique (FDCA) est notamment un produit issu de la biomasse (il peut être obtenu notamment à partir de l'acide mucique ou à partir de l'hydroxyméthylfurfural, qui est obtenu à partir de cellulose). Il est donc a priori un bon candidat comme produit de départ pour préparer le dinitrile correspondant biosourcé.

Le problème est que ce composé (FDCA) est peu soluble dans les solvants classiques et surtout il n'est pas stable thermiquement : il se décarboxyle pour former du furanne qui est un produit chimique cancérogène et/ou mutagène et/ou toxique pour la reproduction (CMR).

Les procédés classiques de préparation de nitrile par nitrilation des acides, qui sont des procédés à haute température et sous pression, ne peuvent donc pas être mis en oeuvre notamment à partir du FDCA. US2005/187266 décrit la synthèse de nitriles à partir des amides correspondants.

On est donc toujours à la recherche d'un procédé de préparation de nitrile, à partir de FDCA ou de dérivés, qui ne présente pas ces inconvénients.

A cet effet, l'invention propose un procédé de préparation d'un composé de formule générale I 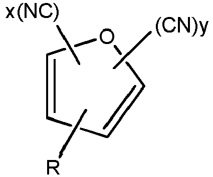 ou d'un composé de formule générale III 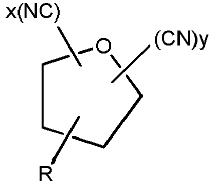 dans lesquelles R symbolise un ou plusieurs éventuels substituants
x, y est égal à 0 ou 1 avec (x+y) est égal à 1 ou 2
Ce procédé consiste à faire réagir de l'ammoniac avec un composé de formule générale II 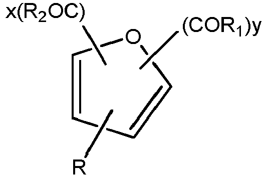 ou avec un composé de formule générale IV 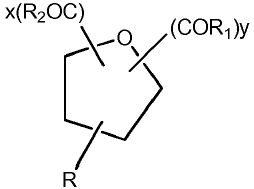 dans lesquelles R₁ et R₂, identiques ou différents représentent -OR₃, R₃ étant un groupe alkyle ayant de 1 à 4 atomes de carbone.
et dans laquelle x, y et R ont la signification indiquée ci-dessus, en présence d'un catalyseur comprenant un orthophosphate de silicium Si₃(PO₄)₄ cristallin.

Le composé de formule générale (I) ou (III) peut être porteur d'un ou plusieurs substituants.

Avantageusement le composé de formule générale II ou IV est issu d'une matière renouvelable d'origine végétale.

Une matière ou ressource renouvelable est une ressource naturelle, animale ou végétale, dont le stock peut se reconstituer sur une période courte à l'échelle humaine. Il faut en particulier que ce stock puisse se renouveler aussi vite qu'il est consommé.

A la différence des matériaux issus de matières fossiles, les matières premières renouvelables contiennent une proportion importante de ¹⁴C. De préférence les nitriles de l'invention sont constitués de carbone organique issu de matières premières renouvelables. Ainsi cette caractéristique préférée pourrait être certifiée par détermination de la teneur en ¹⁴C selon l'une des méthodes décrites dans la norme ASTM D6866, notamment selon la méthode par spectrométrie de masse ou la méthode par spectrométrie à scintillation liquide qui sont décrites dans cette norme.

Ces ressources renouvelables sont généralement produites à partir de matière végétale cultivée ou non telle que les arbres, les plantes comme la canne à sucre, le maïs, le manioc, le blé, le colza, le tournesol la palme, le ricin ou analogues.

Par exemple, le composé de formule générale II ou IV peut être issu de ressources renouvelables telles que les polysaccharides naturels tels que l'amidon ou la cellulose, l'amidon pouvant être extrait, par exemple, du blé, du maïs ou de la pomme de terre. Il peut en particulier provenir de divers procédés de transformation, notamment de procédés chimiques classiques, mais également de procédés de transformation par voie enzymatique ou encore par fermentation.

Par exemple le composé de formule II peut être obtenu à partir d'acide 2,5-furannedicarboxylique, qui est lui-même obtenu notamment à partir de l'acide mucique ou à partir de l'hydroxyméthylfurfural, qui est obtenu à partir de cellulose.

Avantageusement R est choisi parmi :
- les groupes alkyle linéaires ou ramifiés ayant de 1 à 6 atomes de carbone et de préférence de 1 à 4 atomes de carbone,
- les groupes alkyle mono-, poly- ou perhalogénés, linéaires ou ramifiés ayant de 1 à 6 atomes de carbone et de 1 à 13 atomes d'halogène et de préférence de 1 à 4 atomes de carbone et de 1 à 9 atomes d'halogène,
- les groupes éther R₄-O- ou thioéther R₄-S- dans lesquels R₄ représente un groupe alkyle linéaire ou ramifié ayant de 1 à 6 atomes de carbone et encore plus préférentiellement de 1 à 4 atomes de carbone ou le groupe phényle ,
- les groupes acyloxy ou aroyloxy R₄-CO-O- dans lesquels le groupe R₄ a la signification donnée précédemment,
- les groupes acyle ou aroyle R₄-CO- dans lesquels le groupe R₄ a la signification donnée précédemment,
- le groupe hydroxyle,
- un atome d'halogène, de préférence, un atome de fluor.

R1 et R2 sont des groupes -OR₃. Autrement dit le composé de formule (II) ou (IV) est un ester.

Selon un mode de réalisation particulier du procédé de l'invention, x+y est égal à 2 et R1 et R2 sont identiques.

De préférence le composé de formule II ou IV est le 2,5-furanedicarboxylate de méthyle, le 2,5-furanedicarboxylate d'éthyle, le 2-furoate de méthyle ou le 2-furoate d'éthyle.

Conformément au procédé de l'invention, on fait passer un flux gazeux comprenant le composé de formule (II) ou (IV) et l'ammoniac, sur le catalyseur de l'invention.

Bien que la quantité d'ammoniac engagée puisse être égale à la quantité théorique déterminée par la stoechiométrie de la réaction (c'est-à-dire une mole d'ammoniac par mole de fonction ester), il est préférable de conduire la réaction en utilisant un excès d'ammoniac. En général, on préfère utiliser au moins deux moles et plus particulièrement de 2 à 10 moles d'ammoniac par mole de fonction ester. L'excès d'ammoniac présent dans le flux gazeux résultant de la réaction peut être recyclé, après purification.

Le temps de contact apparent du flux gazeux avec le catalyseur défini comme la durée en secondes pendant laquelle une unité de volume de mélange gazeux (mesure dans les conditions normales de pression et de température) est en contact avec l'unité de volume apparent de catalyseur peut être compris entre 0,001 s et 10 min et, de préférence entre 0,01 s et 2 min.

Le procédé est mis en oeuvre généralement sous pression atmosphérique, à une température comprise généralement entre 200 et 600°C, de préférence entre 300°C et 450°C, encore plus préférentiellement entre 350°C et 425°C.

Le composé de formule (II) ou (IV) est généralement vaporisé avant sa mise en contact avec le catalyseur.

Il peut par exemple être vaporisé dans un dispositif de vaporisation ou par pulvérisation dans le flux d'ammoniac surchauffé (300-400°C), généralement après un pré-chauffage. Ces deux modes de vaporisation sont donnés uniquement à titre indicatif.

Le composé de formule (II) ou (IV) est généralement alimenté, avant sa vaporisation, sous forme liquide. Il peut être alimenté sous forme fondue ou sous forme de solution. Il peut s'agir d'une solution aqueuse ou d'une solution dans un solvant.

R₁ et R₂ représentent un groupe -OR₃, c'est-à-dire, le composé de formule (II) ou (IV) est un ester, ce composé peut être sous forme fondue ou sous forme de solution dans un solvant, avantageusement un alcool ou un éther. De préférence on utilise l'alcool qui a été mis en oeuvre lors de la préparation du composé ester de formule (II) ou (IV).
La concentration maximale convenable est fixée par la limite de solubilité du composé de formule II ou IV dans le solvant à la température d'alimentation de la solution dans le dispositif de vaporisation utilisé pour alimenter la solution sur le catalyseur.
A titre d'exemple de solvant, on peut citer par exemple le méthanol, l'éthanol, le THF, le 1,4-dioxanne, le diméthoxyéthane.

Avantageusement, le catalyseur comprend moins de 5% en poids d'orthophosphate de silicium amorphe.

Un catalyseur convenable pour l'invention peut être avantageusement obtenu par imprégnation d'une silice par de l'acide phosphorique en solution aqueuse, puis calcination sous air, pour former l'orthophosphate de silicium. La température de calcination est avantageusement comprise entre 450°C et 800°C, par exemple comprise entre 450°C et 550°C. Un tel procédé de fabrication est notamment décrit dans la demande de brevet français n° 2810317.

Il est également possible d'utiliser des catalyseurs commercialisés par différentes sociétés tels que la société UOP. Toutefois, il peut être nécessaire de traiter ces catalyseurs commerciaux pour augmenter le taux de forme cristalline, par exemple, par un traitement thermique.

Le catalyseur est généralement sous forme solide par exemple en forme de billes, extrudés cylindriques, nid d'abeille ou analogue. Le catalyseur est généralement disposé dans un réacteur sous forme d'un lit fixe à travers duquel est envoyé le composé de formule II ou IV et l'ammoniac sous forme vapeur.

Le catalyseur de l'invention peut contenir également des éléments dopants ou des cocatalyseurs.

Selon une autre caractéristique de l'invention, le catalyseur utilisé pour la mise en oeuvre du procédé de l'invention peut être régénéré par traitement du lit de catalyseur avec de l'air à une température comprise entre 450 et 500°C pendant 10 à 20 heures. Le traitement de régénération peut être contrôlé par détection de la présence de CO₂ dans l'air en sortie de réacteur. Le traitement est arrêté quand l'absence de CO₂ dans l'air est constatée. Le catalyseur ainsi régénéré peut être utilisé pour une nouvelle mise en oeuvre du procédé de l'invention avec des performances catalytiques équivalentes.

Les vapeurs récupérées en sortie de réacteur son condensées pour récupérer le composé comprenant les fonctions nitriles. Ces composés peuvent être ensuite purifiés, par les techniques classiques telles que distillation, cristallisation, extraction ou analogue.

Le catalyseur est avantageusement activé notamment par traitement avec de l'air à une température comprise entre 350°C et 500°C, avant l'alimentation du composé II ou IV.

Selon un mode de réalisation particulier de l'invention, le nitrile de formule (I) ou (III) ainsi récupéré est hydrogéné pour former l'amine correspondante, selon une méthode connue de l'homme du métier. Ainsi on obtient une amine dont tous les carbones sont biosourcés (car issus d'un acide carboxylique biosourcé , c'est-à-dire un acide carboxylique issu d'une matière première renouvelable). Les diamines peuvent être utilisées comme matières premières pour la fabrication de polyamide, qui seront ainsi partiellement ou complètement biosourcés en fonction des acides utilisés pour la polymérisation. Les amines peuvent également être utilisées pour préparer des tensioactifs.

Selon un autre mode de réalisation particulier de l'invention, le nitrile de formule (I) ainsi récupéré est hydrogéné pour former l'amine de formule (V)

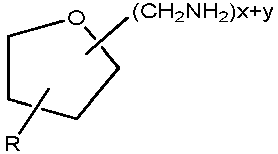

D'autres détails ou avantages de l'invention apparaîtront plus clairement à la vue des exemples donnés ci-dessous.

### EXEMPLES

### Exemple 1

On utilise un réacteur tubulaire en inox de diamètre interne de 2,54 cm équipé d'une sonde de température de 1 mm, chauffé par un four tubulaire électrique. Le lit catalytique composé de 3ml de catalyseur en provenance de la société UOP référencé SPA1 broyé et tamisé entre 2 et 3mm est activé sous un courant d'air de 3 l/h à 450°C pendant 15 heures.

Après cette activation, le lit catalytique est ramené à 400°C et on remplace le courant d'air par un courant d'ammoniac de 2,7l/h. On chauffe alors de nouveau le lit catalytique à 425°C et on injecte sur le lit catalytique une solution méthanolique de 2,5-furannedicarboxylate de méthyle à 20% p/p à un débit de 6ml/h en maintenant le débit d'ammoniac à 2,7l/h. Après 50ml d'injection, le condensat est analysé par CPG. On obtient alors pour une conversion de 38% du diester un rendement de 12% de dicyano 2,5-furanne.

### Exemple 2

On utilise un réacteur tubulaire en inox de diamètre interne de 2,54 cm équipé d'une sonde de température de 1 mm, chauffé par un four tubulaire électrique. Le lit catalytique composé de 3ml de catalyseur en provenance de la société UOP référencé SPA1 broyé et tamisé entre 2 et 3mm est activé sous un courant d'air de 3 l/h à 450°C pendant 15 heures. Après cette activation, le lit catalytique est ramené à 400°C et on remplace le courant d'air par un courant d'ammoniac de 2,7l/h. On chauffe alors de nouveau le lit catalytique à 425°C et on injecte sur le catalyseur une solution éthanolique de 2,5-furannedicarboxylate d'éthyle à 20% poids à un débit de 5ml/h en maintenant le débit d'ammoniac à 2,7l/h.

Après 50ml de solution injectée, on analyse par CPG le condensat. On obtient pour une conversion du diester de 30% un rendement en dinitrile de 15%.

### Exemple 3

On utilise un réacteur tubulaire en inox de diamètre interne de 2,54 cm équipé d'une sonde de température de 1 mm, chauffé par un four tubulaire électrique. Le lit catalytique composé de 3ml de catalyseur en provenance de la société UOP référencé SPA1 broyé et tamisé entre 2 et 3mm est activé sous un courant d'air de 3 l/h à 450°C pendant 15 heures. Après cette activation, le lit catalytique est ramené à 400°C et on remplace le courant d'air par un courant d'ammoniac de 2,4l/h. On chauffe alors de nouveau le lit catalytique à 425°C et on injecte alors sur le catalyseur une solution de 2,5-furannedicarboxylate d'éthyle dans le THF à une concentration p/p de 45% à un débit de 1,5ml/h en maintenant le débit d'ammoniac à 2,4l/h. Après 50ml d'injection, on analyse par CPG le condensat. On obtient pour une conversion du diester de 45% un rendement en dinitrile de 14% et 28% d'ester nitrile.

### Exemple 4 : régénération du catalyseur

Le catalyseur peut, lorsque son activité décroît, être régénéré par un traitement thermique en présence d'air. A partir du lit catalytique froid, on fait passer un courant d'air à un débit de 10l/h et on chauffe progressivement à 500°C en 4 heures. On maintient ensuite dans ces conditions pendant 15 heures. Les gaz sortants du réacteur doivent être exempts de CO2.

La température est ensuite ramenée à 400°C sous 10l/h d'azote et à cette température, le débit d'azote est progressivement remplacé par un débit d'ammoniac. On ne doit pas observer d'exothermie. Le catalyseur est alors régénéré.

## Revendications

1. Procédé de préparation d'un composé de formule générale I 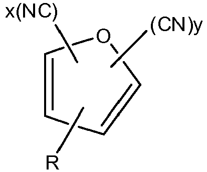 ou respectivement d'un composé de formule générale III 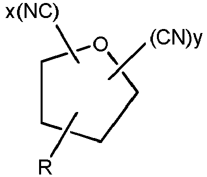 dans lesquelles R symbolise un ou plusieurs éventuels substituants
x, y est égal à 0 ou 1 avec (x+y) est égal à 1 ou 2
consistant à faire réagir, en phase vapeur, l'ammoniac avec un composé de formule générale II 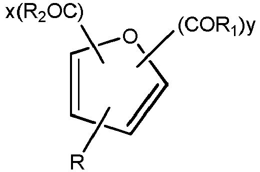 ou respectivement avec un composé de formule générale IV 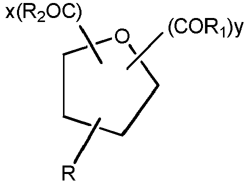 dans lesquelles R₁ et R₂, identiques ou différents représentent -OR₃, R₃ étant un groupe alkyle ayant de 1 à 4 atomes de carbone
en présence d'un catalyseur comprenant un orthophosphate de silicium de formule Si₃(PO₄)₄

2. Procédé selon la revendication 1, **caractérisé en ce que** le composé de formule II et/ou de formule IV est issu d'une matière renouvelable d'origine végétale

3. Procédé selon la revendication 1 ou 2, **caractérisé en ce que** R est choisi parmi :
- les groupes alkyle linéaires ou ramifiés ayant de 1 à 6 atomes de carbone,
- les groupes alkyle mono-, poly- ou perhalogénés, linéaires ou ramifiés ayant de 1 à 6 atomes de carbone et de 1 à 13 atomes d'halogène,
- les groupes éther R₄-O- ou thioéther R₄-S- dans lesquels R₄ représente un groupe alkyle linéaire ou ramifié ayant de 1 à 6 atomes de carbone ,
- les groupes acyloxy ou aroyloxy R₄-CO-O- dans lesquels le groupe R₄ a la signification donnée précédemment,
- les groupes acyle ou aroyle R₄-CO- dans lesquels le groupe R₄ a la signification donnée précédemment,
- le groupe hydroxyle,
- un atome d'halogène.

4. Procédé selon l'une des revendications précédentes, **caractérisé en ce que** x+y est égal à 2 et R₁ et R₂ sont identiques

5. Procédé selon l'une des revendications précédentes, **caractérisé en ce que** le composé de formule II ou de formule IV est choisi parmi le 2,5-furanedicarboxylate de méthyle, le 2,5-furanedicarboxylate d'éthyle, le 2-furoate de méthyle ou le 2-furoate d'éthyle.

6. Procédé selon l'une des revendications précédentes, **caractérisé en ce que** la réaction est mise en oeuvre à une température comprise entre 300 et 450°C

7. Procédé selon l'une des revendications précédentes, **caractérisé en ce que** la réaction est réalisée dans un réacteur comprenant un lit catalytique fixe.

8. Procédé selon l'une des revendications précédentes, **caractérisé en ce que** le catalyseur est obtenu par imprégantion d'une silice avec de l'acide phosphorique, et calcination sous air.

9. Procédé selon la revendication 8, **caractérisé en ce que** la calcination est réalisée à une température comprise entre 400°C et 800°C.

10. Procédé selon l'une des revendications précédentes, **caractérisé en ce qu'**au moins le nitrile (I) et/ou (III) formé est hydrogéné pour former l'amine correspondante.

11. Procédé selon l'une des revendications 1 à 9, **caractérisé en ce qu'**au moins le nitrile (I) formé est hydrogéné pour former l'amine de formule générale (V)

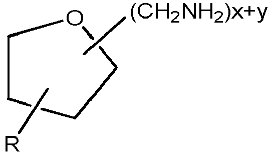

## Patentansprüche

1. Verfahren zur Herstellung einer Verbindung der allgemeinen Formel I 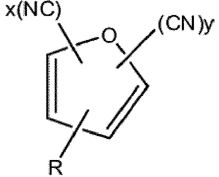 bzw. einer Verbindung der allgemeinen Formel III 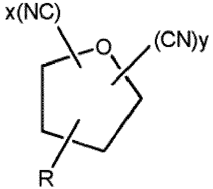 in denen R einen oder mehrere fakultative Substituenten symbolisiert,
x, y gleich 0 oder 1 ist, wobei (x + y) gleich 1 oder 2 ist,
das darin besteht, dass man in der Gasphase Ammoniak in Gegenwart eines Katalysators, der ein Siliciumorthophosphat der Formel Si₃(PO₄)₄ umfasst, mit einer Verbindung der allgemeinen Formel II 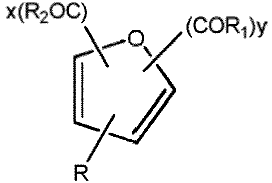 bzw. mit einer Verbindung der allgemeinen Formel IV 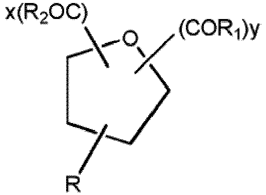 in denen R₁ und R₂, die gleich oder verschieden sind, für -OR₃ stehen, wobei R₃ eine Alkylgruppe mit 1 bis 4 Kohlenstoffatomen bedeutet, umsetzt.

2. Verfahren nach Anspruch 1, **dadurch gekennzeichnet, dass** die Verbindung der Formel II und/oder der Formel IV aus einem erneuerbaren Stoff pflanzlichen Ursprungs stammt.

3. Verfahren nach Anspruch 1 oder 2, **dadurch gekennzeichnet, dass** R aus
- linearen oder verzweigten Alkylgruppen mit 1 bis 6 Kohlenstoffatomen,
- linearen oder verzweigten mono-, poly- oder perhalogenierten Alkylgruppen mit 1 bis 6 Kohlenstoffatomen und 1 bis 13 Halogenatomen,
- Ethergruppen R₄-O- oder Thioethergruppen R₄-S-, in denen R₄ für eine lineare oder verzweigte Alkylgruppe mit 1 bis 6 Kohlenstoffatomen steht,
- Acyloxy- oder Aroyloxygruppen R₄-CO-O-, in denen die Gruppe R₄ die oben angegebene Bedeutung hat,
- Acyl- oder Aroylgruppen R₄-CO-, in denen die Gruppe R₄ die oben angegebene Bedeutung hat,
- der Hydroxylgruppe und
- einem Halogenatom
ausgewählt ist.

4. Verfahren nach einem der vorhergehenden Ansprüche, **dadurch gekennzeichnet, dass** x + y gleich 2 ist und R₁ und R₂ gleich sind.

5. Verfahren nach einem der vorhergehenden Ansprüche, **dadurch gekennzeichnet, dass** die Verbindung der Formel II oder der Formel IV aus 2,5-Furandicarbonsäuremethylester, 2,5-Furandicarbonsäure-ethylester, Furan-2-carbonsäuremethylester oder Furan-2-carbonsäureethylester ausgewählt wird.

6. Verfahren nach einem der vorhergehenden Ansprüche, **dadurch gekennzeichnet, dass** die Umsetzung bei einer Temperatur zwischen 300 und 450°C durchgeführt wird.

7. Verfahren nach einem der vorhergehenden Ansprüche, **dadurch gekennzeichnet, dass** die Umsetzung in einem Reaktor mit einem Katalysatorfestbett durchgeführt wird.

8. Verfahren nach einem der vorhergehenden Ansprüche, **dadurch gekennzeichnet, dass** der Katalysator durch Imprägnieren eines Siliciumdioxids mit Phosphorsäure und Calcinieren an der Luft erhalten wird.

9. Verfahren nach Anspruch 8, **dadurch gekennzeichnet, dass** die Calcinierung bei einer Temperatur zwischen 400°C und 800°C durchgeführt wird.

10. Verfahren nach einem der vorhergehenden Ansprüche, **dadurch gekennzeichnet, dass** mindestens das gebildete Nitril (I) und/oder (III) zu dem entsprechenden Amin hydriert wird.

11. Verfahren nach einem der Ansprüche 1 bis 9, **dadurch gekennzeichnet, dass** mindestens das gebildete Nitril (I) zu dem Amin der allgemeinen Formel (V) 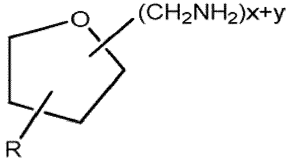 hydriert wird.

## Claims

1. Process for the preparation of a compound of general formula (I): 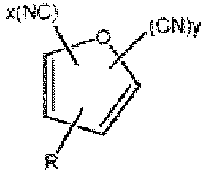 or respectively of a compound of general formula (III) : 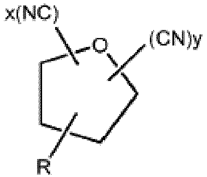
in which R symbolizes one or more optional substituents,
x, y is equal to 0 or 1 with (x+y) equal to 1 or 2,
which consists in reacting, in the vapor phase, ammonia with a compound of general formula (II): 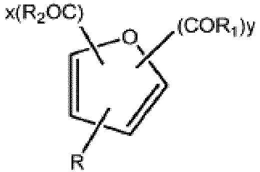 or respectively with a compound of general formula (IV) : 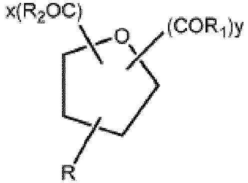
in which R₁ and R₂, which are identical or different, represent-OR₃, R₃ being an alkyl group having from 1 to 4 carbon atoms,
in the presence of a catalyst comprising a silicon orthophosphate of formula Si₃(PO₄)₄.

2. Process according to Claim 1, **characterized in that** the compound of formula (II) and/or of formula (IV) results from a renewable material of vegetable origin.

3. Process according to Claim 1 or 2, **characterized in that** R is chosen from:
- linear or branched alkyl groups having from 1 to 6 carbon atoms,
- linear or branched mono-, poly- or perhalogenated alkyl groups having from 1 to 6 carbon atoms and from 1 to 13 halogen atoms,
- ether R₄-O- or thioether R₄-S- groups in which R₄ represents a linear or branched alkyl group having from 1 to 6 carbon atoms,
- acyloxy or aroyloxy R₄-CO-O- groups in which the R₄ group has the meaning given above,
- acyl or aroyl R₄-CO- groups in which the R₄ group has the meaning given above,
- the hydroxyl group,
- a halogen atom.

4. Process according to one of the preceding claims, **characterized in that** x+y is equal to 2 and R₁ and R₂ are identical.

5. Process according to one of the preceding claims, **characterized in that** the compound of formula (II) or of formula (IV) is chosen from methyl 2,5-furandicarboxylate, ethyl 2,5-furandicarboxylate, methyl 2-furoate or ethyl 2-furoate.

6. Process according to one of the preceding claims, **characterized in that** the reaction is carried out at a temperature of between 300 and 450°C.

7. Process according to one of the preceding claims, **characterized in that** the reaction is carried out in a reactor comprising a fixed catalytic bed.

8. Process according to one of the preceding claims, **characterized in that** the catalyst is obtained by impregnating a silica with phosphoric acid and calcination under air.

9. Process according to Claim 8, **characterized in that** the calcination is carried out at a temperature of between 400°C and 800°C.

10. Process according to one of the preceding claims, **characterized in that** at least the nitrile (I) and/or (III) formed is hydrogenated in order to form the corresponding amine.

11. Process according to one of Claims 1 to 9, **characterized in that** at least the nitrile (I) formed is hydrogenated in order to form the amine of general formula (V):
